(19) Europäisches Patentamt
European Patent Office
Office européen des brevets

(11) EP 1 099 437 A1

(12) DEMANDE DE BREVET EUROPEEN

(43) Date de publication:
**16.05.2001 Bulletin 2001/20**

(51) Int Cl.[7]: **A61K 7/48**

(21) Numéro de dépôt: **00403044.1**

(22) Date de dépôt: **02.11.2000**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **12.11.1999 FR 9914225**

(71) Demandeur: **L'OREAL**
**75008 Paris (FR)**

(72) Inventeurs:
- **Lemann, Patricia**
**94000 Creteil (FR)**
- **Collette, Annick**
**94100 Saint-Maur des Fosses (FR)**
- **Simon, Jean-Christophe**
**75012 Paris (FR)**

(74) Mandataire: **Doressamy, Clarisse**
**L'Oreal,**
**D.P.I.,**
**6, rue Bertrand Sincholle**
**92585 Clichy Cedex (FR)**

# (54) Composition comprenant une substance fluorescente et une charge particuliere, utilisations

(57) La présente demande concerne des compositions cosmétiques, en particulier de maquillage ou de soin de la peau comprenant i) au moins une phase grasse comprenant au moins une partie non volatile et ii) au moins une substance fluorescente, ladite composition étant caractérisée en ce qu'elle comprend iii) au moins une charge ayant des propriétés «soft-focus», le rapport de iii) sur i) étant dans une fourchette donnée.

Les compositions cosmétiques contenant cette association sont particulièrement efficaces pour éclairer le teint et présentent un effet enlumineur de la peau. De plus, elles sont douces à l'application, facilement étalables, non-collantes et ne sont pas desséchantes pour la peau.

EP 1 099 437 A1

## Description

L'invention concerne une composition cosmétique, en particulier de maquillage et/ou de soin, particulièrement efficace pour éclairer le teint. Ces compositions peuvent se présenter en particulier sous la forme de fond de teint.

Les compositions de fonds de teint ont généralement pour objet d'unifier le teint : pour cela, on utilise des compositions matifiantes qui peuvent aider à estomper les défauts de la peau tels que les microreliefs, les rides, les ridules, les pores ou les variations de couleur. Ces compositions matifiantes peuvent aussi être utilisées pour résoudre les problèmes de brillance occasionnés par un excès de sébum et pour améliorer la tenue du maquillage à long terme qui a tendance à se dégrader visuellement au cours de la journée. Elles donnent un aspect mat à la peau résultant d'un pouvoir diffusant de la lumière à la surface de la peau.

Les compositions classiques dites matifiantes contiennent généralement assez peu de corps gras. Elles sont généralement constituées de poudres adsorbantes du sébum et d'huile excédentaire de la composition non-adsorbée par la peau. Parmi les poudres matifiantes d'origine naturelle ou synthétique, on peut citer notamment les charges telles que le talc, l'amidon, le mica , la silice, les poudres de nylon, les poudres de polyéthylène, la poly-beta-alanine, les poly(méthacrylate de méthyle). Ce type de charges présente l'inconvénient de ne pas apporter à la peau un aspect naturel en donnant un aspect poudreux voire plâtreux et d'accentuer les défauts de la peau. De plus, les compositions les contenant sont généralement desséchantes à long terme et s'étalent difficilement. Leur effet matifiant est peu durable dans le temps.

On connaît également dans la demande EP-A-052 769 des compositions matifiantes apportant une couche translucide et un aspect naturel à la peau maquillée. Il s'agit de dispersions de particules sphériques dans un liant gras dans un rapport en poids charges/liant très spécifique. Ces compositions peuvent être desséchantes ; elles ont tendance à pelucher lors de l'étalement et à donner un effet blanchissant à la peau en raison d'une forte concentration en poudre.

Il subsiste donc le besoin de compositions cosmétiques et/ou dermatologiques permettant d'obtenir un teint uniforme, homogène, d'aspect naturel, lumineux, vivant, ces compositions présentant par ailleurs un bon confort après application sur la peau. On recherche également un meilleur effet soft-focus, autrement dit, un effet flou qui camoufle les microreliefs de la peau.

La demande de brevet française publiée sous le numéro FR-2 741 261 décrit des compositions cosmétiques comprenant un agent fluorescent d'avivage également connu comme azurant optique. Ces agents présentent l'avantage d'intensifier l'éclat et d'aviver les teintes des compositions cosmétiques les comprenant à l'application sur la peau ou les cheveux.

La Demanderesse a découvert de manière surprenante qu'en associant à une substance fluorescente une charge présentant des propriétés particulières de «soft-focus», dans une composition donnée, il était possible d'obtenir des compositions dont la capacité à éclairer le teint et l'effet soft focus étaient améliorés.

L'invention a pour objet une composition cosmétique, en particulier de maquillage et/ou de soin, comprenant au moins une phase grasse comprenant une fraction non volatile et au moins une charge «soft-focus», ladite composition étant caractérisée en ce qu'elle comprend au moins une substance fluorescente et en ce que le rapport en poids de la charge «soft-focus» sur la fraction non volatile de la phase grasse est compris entre 30 :70 et 50 :50, de préférence entre 40 :60 et 50 :50.

Une telle composition confère au teint une plus grande uniformité, une plus grande homogénéité, de la transparence, un teint de porcelaine, lumineux, vivant.

Une telle composition se présente généralement sous forme fluide ou solide, à l'exclusion des formes poudres (généralement libres ou compactées). Par suite une telle composition peut se présenter sous forme fluide, sous forme pâteuse, sous forme semi-solide, et sous forme solide, généralement en stick, à l'exclusion des formes poudres. Une telle composition peut se présenter sous la forme d'un fond de teint, par exemple sous forme fluide, en pot ou en stick, de laque à lèvres, typiquement sous forme pâteuse, ou de rouges à lèvres, typiquement sous forme semi-solide (« gloss » en terminologie anglo-saxonne, ou de rouges à lèvres sous forme solide, en stick.

La composition selon l'invention comprend une phase grasse comprenant une fraction non volatile.

Cette fraction non volatile peut comprendre tous corps gras non volatils habituellement utilisés en cosmétique tels que les huiles non volatiles, les cires, les gommes, les résines et/ou les corps gras pâteux d'origine animale, végétale, minérale ou synthétique, et/ou leurs mélanges.

Par huile non volatile, on entend un corps gras liquide à température ambiante (20°C) et qui ne s'évapore pas à cette même température.

Parmi les huiles non volatiles, on peut citer :

- les polyalkyl($C_1$-$C_{20}$) siloxanes et notamment ceux à groupements terminaux triméthylsilyle, de préférence ceux dont la viscosité est inférieure à 0,06 $m^2/s$ parmi lesquels on peut citer les polydiméthylsiloxanes linéaires et les alkylméthylpolysiloxanes tels que la cétyldiméthicone (nom CTFA),

- les silicones modifiées par des groupements aliphatiques et/ou aromatiques, éventuellement fluorés, ou par des groupements fonctionnels tels que des groupements hydroxyles, thiols et/ou amines.

- les huiles de silicone phénylées, notamment celles de formule :

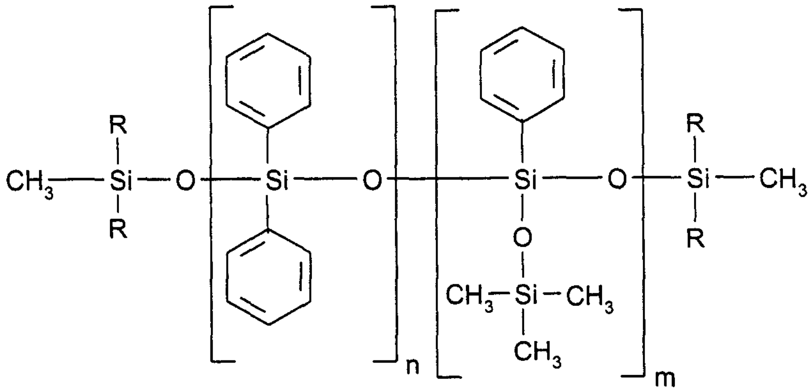

(I)

dans laquelle R est un radical alkyle en C1-C30, un radical aryle ou un radical aralkyle, n est un nombre entier compris entre 0 et 100, et m est un nombre entier compris entre 0 et 100, sous réserve que la somme est comprise entre 1 et 100;

- les huiles d'origine animale, végétale ou minérale, telles que l'huile de paraffine, de vaseline, le perhydrosqualène, la lanoline, l'huile d'abricot, l'huile de germes de blé, d'amande douce, de calophyllum, de sésame, de macadamia, de pépins de raisin, de colza, de coprah, d'arachide, de tournesol, de palme, de ricin, d'avocat, de jojoba, d'olive ou de germes de céréales, la fraction liquide du beurre de karité; des esters d'acides gras et de polyol, en particulier les triglycérides liquides, le myristate d'isopropyle, l'huile de mygliol, le néopentanoate d'isostéaryle, le palmitate d'éthyl-2-hexyle, l'huile de ricin, l'acétyl-citrate de tri-butyle; des alcools, en particulier l'octyl-2-dodecanol ; des acétylglycérides ; des octanoates, décanoates ou ricinoléates d'alcools ou de polyalcools ; des triglycérides d'acides gras; des glycérides, les huiles fluorées et perfluorées ;

- les composés amidés, en particulier ceux décrits dans la demande PCT/FR98/01077 comme le N-néopentanoyl-2-octyl-dodécylamine, le N-néopentanoyl-2-butyl-octylamine, le N-(3,5,5-triméthyl-hexanoyl)-2-octyl-dodécylamine, le N-(3,5,5-triméthyl-hexanoyl)-2-butyl-octylamine,

- leurs mélanges,

De préférence, la fraction non volatile de la phase grasse de la composition selon l'invention comprend une ou plusieurs huiles choisies parmi l'huile de sésame, la lanoline, le myristate d'isopropyle, l'huile de mygliol, le néopentanoate d'isostéaryle, le palmitate d'éthyl-2-hexyle, l'huile de ricin, l'acétyl-citrate de tri-butyle, l'octyl-2-dodecanol, le N-pentanoyl-2-octyl dodecylamine, les polydiméthylsiloxanes et/ou leurs mélanges.

De préférence, la fraction non volatile de la phase grasse est présente dans la composition selon l'invention à une teneur pouvant aller de 1 à 85%, de préférence encore de 1% à 30%, en poids, par rapport au poids total de la composition.

Parmi les cires, on peut notamment citer :

- les cires d'origine animale, végétale, minérale ou synthétique telles que les cires microcristallines, la paraffine, le pétrolatum, la vaseline, l'ozokérite, la cire de Montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25 °C, les ozokérites, les esters gras et les glycérides concrets à 25 °C; les cires de polyéthylène et les cires obtenues par synthèse de Fischer-Tropsch; des huiles hydrogénées concrètes à 25 °C; des lanolines; des esters gras concrets à 25 °C; les cires de silicone; les cires fluorées; leurs mélanges.

On peut définir les composés gras pâteux à l'aide d'au moins une des propriétés physico-chimiques suivantes :

- une viscosité de 0,1 à 40 Pa.s (1 à 400 poises), de préférence 0,5 à 25 Pa.s, mesurée à 40°C avec un viscosimètre rotatif CONTRAVES TV équipé d'un mobile MS-r3 ou MS-r4 à la fréquence de 60 Hz,
- un point de fusion de 25-70°C, de préférence 25-55°C.

Les compositions de l'invention peuvent également comprendre des alkyl, alcoxy ou phényl-diméthicones tels que, par exemple le produit vendu sous la dénomination de "Abil wax 2440" par la Société GOLDSCHMIDT.

Les compositions selon l'invention peuvent également comprendre des résines de silicone comprenant une combinaison des unités $R_3SiO_{1/2}$, $R_2SiO_{2/2}$, $RSiO_{3/2}$ et $SiO_{4/2}$, dans lesquelles R désigne un radical alkyle ayant de 1 à 6 atomes de carbone.

On désigne par $n_1$ l'indice de réfraction moyen de la fraction non volatile de la phase grasse, cet indice de réfraction moyen correspondant à la valeur spectrale moyenne de l'indice pour les longueurs d'onde de la lumière visible, soit les longueurs d'onde allant de 380 à 700 nm. Cette mesure peut être effectuée de façon classique à l'aide d'un réfractomètre.

Les compositions selon l'invention comprennent également au moins une charge particulière dite « soft-focus ».

Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Par charge « soft-focus », on entend une charge qui en plus donne de la transparence au teint et un effet flou.

Cet effet soft-focus est lié à la réflectance spectrale de la charge. Pour une composition échantillon A donnée, il peut être défini par le rapport Th/Td, mesuré à l'aide d'un spectrophotomètre et d'une sphère d'intégration par exemple placée derrière l'échantillon selon le protocole décrit dans l'exemple 1 de la présente demande, et dans lequel :

- Th est la transmittance hémisphérique de la composition échantillon : elle est définie par le rapport entre l'intensité lumineuse reçue par l'échantillon A et l'intensité lumineuse restituée par cet échantillon dans toutes les directions,

- Td est la transmittance directe de la composition échantillon : elle est définie par le rapport entre l'intensité lumineuse reçue par l'échantillon et l'intensité lumineuse restituée par cet échantillon dans le même axe.

Pour des compositions cosmétiques classiques ne présentant pas d'effet soft-focus, ce rapport Th/Td est généralement très inférieur à 2. Les compositions selon l'invention ont de préférence un effet soft-focus, c'est-à-dire un rapport Th/Td mesuré comme dans l'exemple 1 de la présente demande, allant de 1 à 3, de préférence encore allant de 1,7 à 2,3, et ce pour une valeur de Th proche de 100%, c'est-à-dire pouvant aller de 70% à 100%.

La transparence d'une composition donnée ou encore son effet soft-focus peut également être mesurée par colorimétrie dans le système (L, a, b) dans lequel L représente la luminance, a représente l'axe rouge-vert (-a = vert, +a = rouge) et b représente l'axe jaune-bleu (-b =bleu, +b =jaune). ΔE, qui mesure la variation de couleur totale ΔE, est calculé à partir des variations ΔL, Δa et Δb selon la formule suivante :

$$\Delta E = \sqrt{(\Delta L)^2+(\Delta a)^2+(\Delta b)^2}$$

Plus le ΔE est élevé, plus la composition est transparente et meilleur est son effet soft-focus. Les compositions selon l'invention présentent de préférence une valeur de ΔE supérieure ou égale à 30, de préférence encore supérieure ou égale à 45.

On désigne également par $n_2$ l'indice de réfraction moyen de l'ensemble des charges «soft-focus», cet indice de réfraction moyen correspondant à la valeur spectrale moyenne de l'indice pour les longueurs d'onde de la lumière visible, soit les longueurs d'onde allant de 380 à 700 nm. Cette valeur de $n_2$ est généralement décrite dans des ouvrages tel que le « Handbook of Optical Constants of Solids », Edward D. Palik, 1985, éditions Academic Press.

De préférence, dans les compositions selon l'invention, on a :

$$0<|n_1 - n_2| \leq 0{,}3$$

et de préférence encore :

$$0<|n_1 - n_2| \leq 0{,}15.$$

De préférence, au sens de la présente invention, une charge « soft-focus » est une charge choisie parmi les

charges conférant à la composition au moins l'une des propriétés suivantes :

- 1°) le rapport Th/Td, mesuré comme dans l'exemple 1 de la présente demande, va de 1 à 3, de préférence encore va de 1,7 à 2,3, et ce pour une valeur de Th proche de 100%, c'est-à-dire pouvant aller de 70% à 100%,
- 2°) $\Delta E$ est supérieur ou égal à 30, de préférence encore supérieur ou égal à 45,
- 3°) $0<|n_1 - n_2| \leq 0{,}3$ et de préférence encore, $0<|n_1 - n_2| \leq 0{,}15$.

De préférence encore, la charge «soft-focus» selon l'invention est choisie parmi les charges conférant à la composition selon l'invention l'ensemble de ces propriétés.
De préférence, dans la présente invention, la charge « soft-focus » est choisie parmi les charges vérifiant la relation suivante :

$$CPVC - 30\%(CPVC) \leq CPV \leq CPVC - 10\%(CPVC)$$

dans laquelle :

$$CPV = \frac{V}{V+V'} \times 100$$

où :

- V est le volume total de la charge,
- V' est le volume de la fraction non volatile de la phase grasse,

et

$$CPVC = \frac{V}{V+Vh} \times 100$$

où :

- Vh est le volume de la fraction non volatile de la phase grasse juste nécessaire pour combler les interstices entre les particules de la charge. Vh est encore appelé la prise d'huile : il peut être mesuré par exemple selon la norme américaine ASTM D281-84.

La CPV est la concentration pigmentaire volumique de la charge.
La CPVC est la concentration pigmentaire volumique critique (CPVC) de la charge.
De préférence, les charges «soft-focus» ont une taille moyenne des particules inférieure ou égale à 15 microns. De préférence encore, ces charges sont non sphériques.
Les charges «soft-focus» peuvent être de toute nature chimique. Elles peuvent ainsi être choisies parmi la silice, le talc, les composites silice/$TiO_2$ ou silice/oxyde de zinc, la poudre de polyéthylène, la poudre d'amidon, la poudre de nylon, les poudres de copolymères styrène/acrylique et/ou leurs mélanges.
On peut ainsi obtenir un fond de teint qui comprend peu de pigments mais qui camoufle bien les micro reliefs de la peau. Cette composition présente des propriétés de soft-focus, autrement dit, lorsqu'on l'applique sur la peau, elle donne un effet flou qui camoufle les micro-reliefs de la peau.
Parmi les charges « soft-focus » convenant particulièrement bien à l'invention, on peut citer le talc de granulométrie (ou taille moyenne des particules) 1,8 micron vendu sous la dénomination commerciale « Talc P3 » par la société Nippon Talc, la poudre de nylon 12 vendue sous la dénomination « Orgasol 2002 Extra D Nat Cos » par la société Atochem, les micro-billes de silice vendues sous la dénomination « SB-700 » ou « SB-150 » par la société Miyoshi.
Dans une forme particulièrement avantageuse de réalisation de l'invention, la charge «soft-focus» est du talc de taille moyenne des particules inférieure ou égale à 3 microns.
De préférence, la charge « soft-focus » est présente dans la composition à une teneur allant de 0,1 à 20%, de préférence encore de 8% à 15%, en poids par rapport au poids total de la composition.
Les compositions selon l'invention comprennent également une substance fluorescente.

Les substances fluorescentes sont bien connues de l'homme du métier. Elles peuvent être des pigments ou des colorants. Par pigments, on entend des particules, minérales ou organiques, insolubles dans la composition. Par colorant, on entend des composés chimiques solubilisés dans la composition. Les colorants peuvent être hydrosolubles ou liposolubles. Les substances fluorescentes sont par exemple décrites dans « Luminescent materials (fluorescent daylight), Encyclopedia of Chemical Technology, Kirk-Othmer", vol 14, p. 546-569, 3ème édition, 1981, Wiley.

Au sens de la présente invention, on entend par substance fluorescente une substance qui, sous l'effet de rayons ultraviolet et/ou de la lumière visible, ré émet dans le visible la portion de lumière qu'elle a absorbé sous la même couleur que celle qu'elle reflète naturellement. La couleur réfléchie naturellement est ainsi renforcée par la couleur ré émise et apparaît extrêmement brillante.

Comme substances fluorescentes inorganiques utilisables dans la présente invention, on peut par exemple citer celles décrites dans la demande JP05-117127 et en particulier les substances fluorescentes inorganiques à base d'oxyde de zinc.

Comme substances fluorescentes organiques utilisables dans la présente invention, on peut citer les pigments fluorescents à la lumière du jour : ces pigments sont généralement fabriqués à partir de colorants fluorescents qui sont préalablement dissous dans une résine support afin d'obtenir une solution solide laquelle est ensuite broyée en une poudre de particules de résine présentant des propriétés fluorescentes. La préparation de tels pigments fluorescents est écrite dans EP 0 370 470, US 2, 851, 424, US 3, 711, 604, US 3, 856, 550 et US 2, 938, 878.

Les pigments fluorescents convenant particulièrement bien à la présente invention peuvent ainsi être choisis parmi les résines colorées de polyamide et/ou de formaldéhyde/benzoguanamine et/ou de melamine/formaldéhyde/sulfonamide, parmi les co-condensats aminotriazine/formaldéhyde/sulfonamide colorés et/ou parmi les paillettes polyester métallisées et/ou leurs mélanges. Ces pigments fluorescents peuvent aussi se présenter sous la forme de dispersions aqueuses de pigments fluorescents.

Comme pigments fluorescents convenant particulièrement bien à la présente invention, on peut citer le co-condensat aminotriazine/formaldehyde/sulfonamide fluorescent coloré en ros de taille moyenne des particules 3-4 microns vendu sous la dénomination commerciale « Fiesta Astral Pink FEX-1 » et le co-condensat aminotriazine/formaldehyde/sulfonamide fluorescent coloré en bleu de taille moyenne des particules 3-4,5 microns vendu sous la dénomination commerciale « Fiesta Comet Blue FTX-60 » par la société Swada ou encore la résine benzoguanamine/formaldéhyde recouverte de résine formaldéhyde/urée et colorée en jaune vendue sous la dénomination commerciale « FB-205 Yellow » et la résine benzoguanamine/formaldéhyde recouverte de résine formaldéhyde/urée et colorée en rouge vendue sous la dénomination commerciale « FB-400 Orange Red » par la société UK SEUNG CHEMICAL, la résine polyamide colorée en orange vendue sous la dénomination commerciale « Flare 911 Orange 4 » par la société Sterling Industrial Colors.

Lorsque les substances fluorescentes organiques sont blanches, on les appelle également des azurants optiques.

Les azurants optiques sont des composés bien connus de l'homme du métier. De tels composés sont décrits dans "Fluorescent Whitening Agent, Encyclopedia of Chemical Technology, Kirk-Othmer", vol 11, p. 227-241, 4ème édition, 1994, Wiley.

On peut plus particulièrement les définir comme des composés qui absorbent essentiellement dans l'UVA entre 300 et 390 nm et réémettent essentiellement entre 400 et 525 nm.

Parmi les azurants optiques, on peut plus particulièrement citer les dérivés du stilbène, en particulier les polystyrylstilbènes et les triazinstilbènes, les dérivés coumariniques, en particulier les hydroxycoumarines et les aminocoumarines, les dérivés oxazole, benzoxazole, imidazole, triazole, pyrazoline, les dérivés du pyrène et les dérivés de porphyrine et/ou leurs mélanges.

De tels composés sont facilement disponibles commercialement. On peut citer par exemple :

- le dérivé stilbénique de naphto-triazole vendu sous la dénomination commerciale « Tinopal GS », le di-styryl-4,4' biphényle sulfonate di-sodique (nom CTFA : disodium distyrylbiphenyl disulfonate) vendu sous la dénomination commerciale « Tinopal CBS-X », le dérivé cationique d'aminocoumarine vendu sous la dénomination commerciale « Tinopal SWN CONC. », le 4,4'-bis[(4,6-dianilino-1,3,5-triazin-2-yl)amino]stilbène-2,2'-disulfonate de sodium vendu sous la dénomination commerciale « Tinopal SOP », le 4,4'-bis-[(4-anilino-6-bis(2-hydroxyéthyl)amino-1,3,5-triazin-2-yl)amino]stilbène-2,2'-disulfonic acide vendu sous la dénomination commerciale « Tinopal UNPA-GX », le 4,4'-bis-[anilino-6-morpholine-1,3,5-triazin-2-yl)amino]stilbène vendu sous la dénomination commerciale « Tinopal AMS-GX », le 4,4'-bis-[(4-anilino-6-(2-hydroxyéthyl)méthyl amino-1,3,5-triazin-2-yl)amino]stilbène-2,2'-disodium sulfonate vendu sous la dénomination commerciale « Tinopal 5BM-GX », tous par la société CIBA Spécialités Chimiques,
- le 2,5 thiophène di-yl bis(5 ter-butyl-1,3 benzoxazole) vendu sous la dénomination commerciale « Uvitex OB » par la société CIBA,
- le dérivé anionique du di-aminostilbène en dispersion dans l'eau vendu sous la dénomination commerciale « Leucophor BSB liquide » par la société CLARIANT,

- les laques d'azurant optique vendues sous la dénomination commerciale « COVAZUR » par la société WACKHERR.

Les azurants optiques utilisables dans la présente invention peuvent aussi se présenter sous la forme de copolymères, par exemple d'acrylates et/ou de méthacrylates, greffés par des groupement azurants optiques comme décrits dans la demande FR99-10942.

Les azurants optiques préférentiellement utilisés selon l'invention sont le 4,4'-bis[(4,6-dianilino-1,3,5-triazin-2-yl)amino]stilbène-2,2'-disulfonate de sodium, le 2,5 thiophène di-yl bis(5 ter-butyl-1,3 benzoxazole), le di-styryl-4,4' biphényle sulfonate di-sodique et/ou leurs mélanges.

Dans la présente invention, de préférence, les substances fluorescentes sont choisies parmi les pigments fluorescents organiques.

Les substances fluorescentes sont de préférence présentes dans la composition, à une teneur allant de 0,1 à 20%, de préférence de 0,1 à 15%, de préférence encore de 0,5 à 3% en poids, par rapport au poids total de la composition.

La phase grasse des compositions selon l'invention peut éventuellement comprendre en outre des huiles volatiles.

On entend par huile volatile, tout composé susceptible de s'évaporer au contact de la peau. De préférence, on utilise des huiles dont le point éclair est suffisamment élevé pour permettre l'utilisation de ces huiles en formulation.

Ces composés volatils peuvent être choisis en particulier parmi les huiles hydrocarbonées et/ou les huiles siliconées, cycliques ou linéaires, seules ou en mélange. On peut ainsi citer les huiles siliconées volatiles, telles que :

- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,

- les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,

- les silicones volatiles linéaires ayant de 2 à 9 atomes de silicium. Il s'agit par exemple de l'hexaméthyldisiloxane ou un PDMS de faible viscosité (1 cSt). On peut encore citer les alkyltrisiloxanes tels que l'hexylheptaméthyltrisiloxane ou l'octylheptaméthyltrisiloxane.

On peut également citer les huiles volatiles hydrocarbonées, telles que les isoparaffines et notamment l'isododécane.

Les compositions selon l'invention peuvent également comprendre d'autres pigments et/ou charges que ceux cités ci-dessus, c'est-à-dire des pigments non fluorescents et/ou des charges ne présentant pas de propriété soft-focus.

On peut citer, parmi les pigments minéraux non fluorescents, les dioxydes de titane, de zirconium ou de cérium, ainsi que les oxydes de zinc, de fer ou de chrome, le bleu ferrique, les nacres telles que le mica recouvert d'oxyde de titane, d'oxyde de fer, de pigment naturel ou d'oxychlorure de bismuth ainsi que le mica titane coloré.

Les pigments minéraux non fluorescents peuvent être présents dans les compositions selon l'invention à une teneur pouvant aller jusqu'à 10% en poids, par rapport au poids total de la composition. Pour un meilleur effet soft-focus, les pigments minéraux sont de préférence présents à une teneur inférieure ou égale à 5% en poids, de préférence encore inférieure ou égale à 2,5% en poids, par rapport au poids total de la composition.

Parmi les pigments organiques, on peut citer le noir de carbone, et les laques couramment employées pour conférer aux lèvres et à la peau un effet de maquillage, qui sont des sels de calcium, de baryum, d'aluminium ou de zirconium, de colorants acides tels que les colorants halogéno-acides, azoïques ou anthraquinoniques.

Ces pigments peuvent être traités de manière à rendre leur surface hydrophobe; ce traitement peut être effectué selon les méthodes connues de l'homme du métier; les pigments peuvent notamment être enrobés par des composés siliconés tels que des PDMS et/ou par des polymères.

Les pigments non fluorescents peuvent être présents dans la composition de l'invention à une teneur allant de 0 à 10 %, en poids par rapport au poids total de la composition. Toutefois, dans une forme préférée de réalisation de l'invention, la composition est exempte de pigments non fluorescents.

Parmi les charges, on peut citer toutes les charges, minérales ou de synthèse, lamellaires ou non lamellaires, classiquement utilisées dans le domaine cosmétique et qui ne présentent pas de propriétés soft-focus. On peut citer le talc, le mica, la silice, le kaolin, les poudres de Nylon et de polyéthylène, le Téflon, l'amidon, le nitrure de bore, les microsphères telles que l'Expancel (Nobel Industrie), les micro-éponges comme le Polytrap (Dow Corning) et les microbilles de résine de silicone (Tospearls de Toshiba, par exemple).

Ces charges ne présentant pas de propriété soft-focus peuvent être présentes dans la composition à raison

de 0-20 % en poids, de préférence de 2-15 % en poids, par rapport au poids total de la composition.

Les compositions selon l'invention peuvent également comprendre des nacres. Par nacres, il faut comprendre des particules irisées qui réfléchissent la lumière.

Les nacres peuvent être présentes dans la composition à raison de 0-20 % en poids, de préférence de 2-15 % en poids, par rapport au poids total de la composition. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Les compositions selon l'invention peuvent également comprendre d'autres colorants que ceux déjà cités comme par exemple les colorants hydrosolubles ou liposolubles choisis parmi les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le Rouge Soudan III (nom CTFA D&C red 17), la lutéine, le vert de quinizarine (nom CTFA DC green 6), le pourpre d'Alizurol SS (nom CTFA DC violet N°2), les dérivés caroténoïdes comme le lycopène, le béta-carotène, la bixine, la capsantéine, et/ou leurs mélanges.

Les compositions de l'invention comprennent en outre un milieu cosmétiquement et/ou physiologiquement acceptable, c'est-à-dire un milieu compatible avec la peau, autrement dit toute zone cutanée du corps et du visage.

La composition selon l'invention peut éventuellement comprendre une phase aqueuse qui peut comprendre de l'eau, une eau florale telle que l'eau de bleuet et/ou une eau minérale telle que l'eau de VITTEL, l'eau de LUCAS ou l'eau de LA ROCHE POSAY et/ou une eau thermale.

Les compositions de l'invention peuvent ainsi se présenter sous la forme d'une composition anhydre ou d'une émulsion huile-dans-eau (H/E), une émulsion eau-dans-huile (E/H), une émulsion multiple ou une solution multiphasée. Elles peuvent également se présenter sous la forme d'une dispersion vésiculaire, par exemple sous la forme d'une phase huileuse dispersée dans une phase aqueuse et stabilisée par des liposomes.

Dans le cas où la composition selon l'invention est une émulsion, elle peut comprendre des composés amphiphiles c'est-à-dire des composés comprenant à la fois une partie lipophile (partie apolaire) et une partie hydrophile (partie polaire) et pouvant s'adsorber à une surface ou une interface. De tels composés sont par exemple les tensioactifs et les co-tensioactifs.

Comme tensioactif H/E, on peut citer notamment (CTFA) : l'acide stéarique combiné à la triéthanolamine, le mélange de mono et distéarate de glycérol, le lauroyl sarcosinate de sodium, le cétéaryl-glucoside, le PEG-40 stéarate, le sorbitan tristéarate, le sorbitan stéarate, le polysorbate 60, le mélange sorbitan stéarate/sucrose cocoate, le mélange de glycéryl stéarate/PEG-100 stéarate, le PEG-400, le stéarate de glycéryle, le mélange de PEG-6/PEG-32/glycol stéarate. Comme tensioactif E/H, on peut citer notamment le mélange polyglycéryl-4 isostéarate/ cétyldiméthicone copolyol/ hexyl laurate et le mélange Minéral oil/petrolatum/ozokérite/glycéryl oléate/alcool de lanoline.

Lorsque la composition selon l'invention comprend des tensio-actifs, ceux-ci font partie de la fraction non volatile de la phase grasse et sont donc pris en compte dans le calcul de $n_1$.

De préférence, la composition selon l'invention se présente sous la forme d'une émulsion E/H.

La composition selon l'invention peut comprendre en outre tout additif usuellement utilisé dans le domaine cosmétique, tels que des antioxydants, des filtres UV, des colorants, des parfums, des huiles essentielles, des conservateurs, des actifs cosmétiques, des hydratants, des vitamines, des sphingolipides, des polymères liposolubles notamment hydrocarbonés, tels que le polybutène, les polyalkylènes, les polyacrylates et les polymères siliconés compatibles avec les corps gras, et/ou leurs mélanges.

Ces additifs peuvent être présents dans la composition à raison de 0-15% en poids, par rapport au poids total de la composition.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les compositions selon l'invention peuvent se présenter sous la forme d'un produit cosmétique et notamment sous la forme d'un produit de maquillage, en particulier un fond de teint, un anticerne, un produit de maquillage pour le corps ou bien encore d'un produit de soin pour le corps et/ou le visage.

Les compositions peuvent également se présenter sous la forme d'un produit de soin de la peau et/ou du corps, comme une crème ou un gel blanchissants ou comme un produit de soin conférant à la peau uniformité et/ou homogénéité et/ou transparence et/ou aspect de porcelaine.

Les compositions selon l'invention peuvent être préparées selon les méthodes de préparation classiques bien connues de l'homme du métier.

La présente invention a encore pour objet un procédé non thérapeutique de maquillage de la peau, caractérisé par le fait que l'on applique sur la peau une composition telle que définie ci-dessus.

La présente invention a encore pour objet l'utilisation d'au moins une charge «soft-focus» et d'au moins une substance fluorescente dans une composition comprenant au moins une phase grasse comprenant une fraction non

volatile dans le but de conférer à la peau un effet soft-focus amélioré.

La présente invention a encore pour objet l'utilisation d'au moins une charge «soft-focus» et d'au moins une substance fluorescente pour la préparation d'une composition comprenant au moins une phase grasse comprenant une fraction non volatile, dans le but de conférer à la peau un effet soft-focus amélioré.

Les exemples suivants illustrent l'invention. Dans ces exemples, les proportions indiquées sont des pourcentages en poids.

**EXEMPLE 1** :

La Demanderesse a réalisé les compositions de Fond de Teint suivantes :

**Formule :**

*Phase A :*

- mélange de poly diméthylsiloxane à groupements alpha-omega oxyéthyléné oxypropyléné (58/42) et de cyclopentadiméthylsiloxane (85/15) vendu sous la dénomination commerciale « Abil EM 97 » par la société GOLDSCHMIDT 6,64%
- mono-di-glycerides d'acide iso-stéarique estérifiés par de l'acide succinique non stabilisés vendu sous la dénomination commerciale « Imwitor 780 K » par la société HULS 2,21%
- cyclopentadiméthylsiloxane (viscosite: 4 cst) qsp 100%
- polydiméthylsiloxane (viscosité: 10 cst) 4,05%
- iso-dodécane 4,6%

*Phase A1 :*

- cyclopentadiméthylsiloxane (viscosité: 4 cst) 2,7%
- 2,5-thiophenediylbis(5-tert-butyl-1,3-benzoxazole) vendu sous la dénomination commerciale « Uvitex OB » par Ciba X%
- résine polyamide fluorescente colorée en orange vendue sous la dénomination commerciale « Flare 911 Orange 4 » par Sterling Industrial Colors Y%

*Phase A2 :*

- talc de taille moyenne des particules 1,8 micron vendu sous la dénomination commerciale « Talc P3 » par Nippon Talc 8%

*Phase B :*

- eau déminéralisée stérilisée 46,55%
- sulfate de magnésium, 7 $H_2O$ 0,7%
- conservateurs 0,25%

Les valeurs pour X et Y et les résultats obtenus sont rassemblés dans le tableau I suivant :

Tableau I

| Formule | % de X (pigment fluorescent blanc) | % de Y (pigment flurescent orange) | ΔE | Effet soft focus Th/Td |
|---|---|---|---|---|
| Formule n°1 | 0 | 0 | 44,8 | 1,35 |
| Formule n°2 | 0,5% | 0 | 49,6 | 1,6 |
| Formule n°3 | 0 | 1,17 | 64 | 1,5 |
| Formule n°4 | 1,3 | 1,5 | 52,7 | 1,8 |

Dans tous les cas, le rapport en poids de la charge « soft-focus » sur la fraction non volatile de la phase grasse est égal à 0,62.

Mesure de ΔE :

Un film de chaque formule, d'épaisseur contrôlée, à savoir 90 μm, a été étalé sur une plaque de contraste noir/blanc. Les mesures colorimétriques ont été effectuées à l'aide d'un colorimètre CR300 de MINOLTA.

Les résultats sont donnés dans le système (L, a, b) dans lequel L représente la luminance, a représente l'axe rouge-vert (-a = vert, +a = rouge) et b représente l'axe jaune-bleu (-b =bleu, +b =jaune). ΔE, qui mesure la variation de couleur totaleΔE, est calculé à partir des variations ΔL, Δa et Δb selon la formule suivante :

$$\Delta E = \sqrt{(\Delta L)^2+(\Delta a)^2+(\Delta b)^2}$$

Plus le ΔE est élevé, plus la composition est transparente et meilleur est son effet soft-focus.

Mesure de l'effet soft-focus :

Un film de chaque formule, d'épaisseur contrôlée, à savoir 10 μm, a été étalé sur une lame de quartz à creuset.

*a°) mesure de Th :*

Th est la transmittance hémisphérique : elle est définie par le rapport entre l'intensité lumineuse reçue par l'échantillon testé et l'intensité lumineuse transmise par cet échantillon et restituée par cet échantillon dans toutes les directions.

Elle est mesurée à l'aide d'un spectrophotomètre VARIAN Cary 300 et d'une sphère d'intégration de marque Labsphere placée derrière l'échantillon, selon le protocole suivant : on utilise le spectrophotomètre en mode de transmission diffuse, pour un spectre de 400 à 700 nm. On se place en mode transmission %T, à une vitesse de balayage à 240 nm/mn, en « double reverse ». On fait une « baseline correction » en faisant la mesure d'une lame vide de référence. Ceci donne la valeur maximale de l'intensité qui peut être transmise. On place la lame de quartz contenant l'échantillon dans le compartiment de mesure. On mesure Th.

*b°) mesure de Td :*

Td est la transmittance directe : elle est définie par le rapport entre l'intensité lumineuse reçue par l'échantillon et l'intensité lumineuse restituée par cet échantillon dans le même axe. Td est donc une partie de Th.

Elle a été mesurée à l'aide du même spectrophotomètre que pour Th selon le protocole décrit suivant : on utilise le spectrophotomètre en mode de transmission directe, pour un spectre de 400 à 700 nm. On se place en mode transmission %T, à une vitesse de balayage 240 nm, en mode double. On place une lame de quartz vide dans le compartiment de référence et la lame de quartz contenant l'échantillon dans le compartiment de mesure. On mesure Td.

Les quantités Th et Td sont les moyennes des valeurs spectrales mesurées.

Plus le rapport Th/Td se rapproche d'une valeur comprise entre 1 et 3, tout en tendant vers 2, avec une valeur pour Th proche de 100%, meilleur est l'effet soft-focus et plus transparente est la composition.

Si on compare la formule n° 1 avec la formule n°2, on s'aperçoit que l'on a une valeur de soft-focus plus proche de 2, donc le pigment fluorescent blanc a amélioré l'effet soft-focus. On constate le même phénomène entre la formule n°3 et la formule n°1.

De plus, de façon surprenante, ces formules n°2 et 3 qui contiennent pourtant plus de matière pulvérulente que la formule n°1, sont beaucoup plus transparentes à l'application (elles présentent un ΔE plus élevé). Ainsi, la présence de pigments fluorescents apporte un effet enlumineur qui éclaire le teint.

Le meilleur compromis est obtenu avec la formule n°4 , avec la valeur de Th/Td la plus proche de 2 et un très bon effet enlumineur.

Ainsi pour une quantité de charge «soft-focus» fixée (ici 8% de talc de taille moyenne des particules 1,8 micron), l'ajout de pigments fluorescents permet de manière efficace et surprenante, d'améliorer l'effet soft focus et d'apporter en plus un effet lumineux.

## Revendications

1. Composition cosmétique, en particulier de maquillage et/ou de soin, comprenant au moins une phase grasse comprenant une fraction non volatile, et au moins une substance fluorescente, caractérisée en ce que ladite composition comprend au moins une charge « soft-focus» et en ce que le rapport en poids de la charge « soft-focus» sur la fraction non volatile de la phase grasse est compris entre 30 :70 à 50 :50.

2. Composition selon la revendication 1 telle que la charge « soft-focus » est choisie parmi les charges conférant à la composition au moins l'une des propriétés suivantes :

   - 1°) la composition présente un effet soft-focus Th/Td allant de 1 à 3, de préférence encore allant de 1,7 à 2,3, et ce pour une valeur de Th allant de 70% à 100%, où Th est la transmittance hémisphérique de la composition et Td est la transmittance directe de la composition,

   - 2°) la composition présente un ΔE supérieur ou égal à 30, de préférence encore supérieur ou égal à 45, où

     $$\Delta E = \sqrt{(\Delta L)^2+(\Delta a)^2+(\Delta b)^2}$$

     et L représente la luminance, a représente l'axe rouge-vert (-a = vert, +a = rouge) et b représente l'axe jaune-bleu (-b =bleu, +b =jaune) dans le système (L, a, b),

   - 3°) la composition vérifie la relation suivante :
     $0<|n_1 - n_2| \leq 0,3$ et de préférence encore, $0<|n_1 - n_2| \leq 0,15$,
     dans laquelle $n_1$ est l'indice de réfraction moyen de la fraction non volatile de la phase grasse et $n_2$ est l'indice de réfraction moyen de la charge.

3. Composition selon la revendication 2 telle que la charge « soft-focus » est choisie parmi les charges conférant à la composition l'ensemble des propriétés 1°) à 3°).

4. Composition selon l'une quelconque des revendications 1 à 3 telle qu'elle présente un effet soft-focus Th/Td allant de 1 à 3, de préférence encore allant de 1,7 à 2,3, et ce pour une valeur de Th allant de 70% à 100%, où Th est la transmittance hémisphérique de la composition et Td est la transmittance directe de la composition.

5. Composition selon l'une quelconque des revendications 1 à 3 telle qu'elle présente un ΔE supérieur ou égal à 30, de préférence encore supérieur ou égal à 45,
   où

   $$\Delta E = \sqrt{(\Delta L)^2+(\Delta a)^2+(\Delta b)^2}$$

   et L représente la luminance, a représente l'axe rouge-vert (-a = vert, +a = rouge) et b représente l'axe jaune-bleu

(-b =bleu, +b =jaune) dans le système (L, a, b).

**6.** Composition selon l'une quelconque des revendications 1 à 3 telle qu'elle vérifie la relation suivante : $0<|n_1 - n_2| \leq 0{,}3$ et de préférence encore, $0< |n_1 - n_2| \leq 0{,}15$, dans laquelle $n_1$ est l'indice de réfraction moyen de la fraction non volatile de la phase grasse et $n_2$ est l'indice de réfraction moyen de la charge.

**7.** Composition selon l'une quelconque des revendications précédentes telle que la charge « soft-focus » est choisie parmi les charges vérifiant la relation suivante :

$$CPVC - 30\%(CPVC) \leq CPV \leq CPVC - 10\%(CPVC)$$

dans laquelle :

$$CPV= \frac{V}{V+V'} \times 100$$

où :

- V est le volume total de la charge,
- V' est le volume de la fraction non volatile de la phase grasse, et

$$CPVC= \frac{V}{V+Vh} \times 100$$

où :
- Vh est le volume de la fraction non volatile de la phase grasse juste nécessaire pour combler les interstices entre les particules de la charge.

**8.** Composition selon l'une quelconque des revendications précédentes telle que la charge « soft-focus » a une taille moyenne des particules inférieure ou égale à 15 microns.

**9.** Composition selon l'une quelconque des revendications précédentes telle que la charge « soft-focus » est non sphérique.

**10.** Composition selon l'une quelconque des revendications précédentes telle que la charge « soft-focus » est choisie parmi la silice, le talc, les composites silicel/$TiO_2$ ou silice/oxyde de zinc, la poudre de polyéthylène, la poudre d'amidon, la poudre de nylon, les poudres de copolymères styrène/acrylique et/ou leurs mélanges.

**11.** Composition selon l'une quelconque des revendications précédentes telle que la charge « soft-focus » est du talc de taille moyenne des particules inférieure ou égale à 3 microns.

**12.** Composition selon l'une quelconque des revendications précédentes telle que la charge « soft-focus » est présente dans la composition à une teneur allant de 0,1 à 20%, de préférence encore de 8% à 15%, en poids par rapport au poids total de la composition.

**13.** Composition selon l'une quelconque des revendications précédentes telle que les substances fluorescentes sont choisies parmi les pigments fluorescents organiques.

**14.** Composition selon la revendication 13 telle que lesdits pigments fluorescents sont choisis parmi les résines colorées de polyamide et/ou de formaldéhyde/benzoguanamine et/ou de melamine/formaldéhyde/sulfonamide, parmi les co-condensats aminotriazine/formaldéhyde/sulfonamide colorés et/ou parmi les paillettes polyester métallisées et/ou leurs mélanges.

**15.** Composition selon la revendication 13 telle que les pigments fluorescents sont choisis parmi les dérivés du stilbène, en particulier les polystyrylstilbènes et les triazinstilbènes, les dérivés coumariniques, en particulier les hydroxycoumarines et les aminocoumarines, les dérivés oxazole, benzoxazole, imidazole, triazole, pyrazoline, les dérivés du pyrène et les dérivés de porphyrine et/ou leurs mélanges.

**16.** Composition selon la revendication 15 telle que les pigments fluorescents sont choisis parmi le 4,4'-bis[(4,6-dianilino-1,3,5-triazin-2-yl)amino]stilbène-2,2'-disulfonate de sodium, le 2,5 thiophène di-yl bis(5 ter-butyl-1,3 benzoxazole), le distyryl-4,4' biphényle sulfonate di-sodique et/ou leurs mélanges.

**17.** Composition selon l'une quelconque des revendications précédentes telle que les substances fluorescentes sont présentes dans la composition, à une teneur allant de 0,1 à 20%, de préférence de 0,1 à 15%, de préférence encore de 0,5 à 3%, en poids, par rapport au poids total de la composition.

**18.** Composition selon l'une quelconque des revendications précédentes telle que la fraction non volatile de la phase grasse comprend une ou plusieurs huiles choisies parmi l'huile de sésame, la lanoline, le myristate d'isopropyle, l'huile de mygliol, le néopentanoate d'isostéaryle, le palmitate d'éthyl-2-hexyle, l'huile de ricin, l'acétyl-citrate de tri-butyle, l'octyl-2-dodecanol, le N-pentanoyl-2-octyl dodecylamine, les polydiméthylsiloxanes et/ou leurs mélanges.

**19.** Composition selon l'une quelconque des revendications précédentes telle que la fraction non volatile de la phase grasse est présente dans la composition à une teneur pouvant aller de 1 à 85%, de préférence encore de 1% à 30%, en poids, par rapport au poids total de la composition.

**20.** Composition selon l'une quelconque des revendications précédentes telle que la phase grasse comprend en outre des huiles volatiles.

**21.** Composition selon l'une quelconque des revendications précédentes telle qu'elle comprend en outre des pigments minéraux non fluorescents à une teneur inférieure ou égale à 5% en poids, de préférence inférieure ou égale à 2,5% en poids, par rapport au poids total de la composition.

**22.** Composition selon l'une quelconque des revendications précédentes telle que la composition est exempte de pigments non fluorescents.

**23.** Composition selon l'une quelconque des revendications précédentes telle qu'elle comprend en outre un milieu cosmétiquement et/ou physiologiquement acceptable.

**24.** Composition selon l'une quelconque des revendications précédentes telle qu'elle comprend en outre une phase aqueuse.

**25.** Composition selon l'une quelconque des revendications précédentes telle qu'elle se présente sous la forme d'une émulsion E/H.

**26.** Composition selon l'une quelconque des revendications précédentes telle qu'elle comprend en outre un additif choisi parmi les antioxydants, les filtres UV, les colorants, les parfums, les huiles essentielles, les conservateurs, les actifs cosmétiques, les hydratants, les vitamines, les sphingolipides, les polymères liposolubles notamment hydrocarbonés, tels que le polybutène, les polyalkylènes, les polyacrylates et les polymères siliconés compatibles avec les corps gras.

**27.** Composition selon l'une quelconque des revendications précédentes telle qu'elle se présente sous la forme d'un produit cosmétique et notamment sous la forme d'un produit de maquillage, en particulier un fond de teint, un anticerne, un produit de maquillage pour le corps ou bien encore d'un produit de soin pour le corps et/ou le visage.

**28.** Composition selon l'une quelconque des revendications précédentes telle qu'elle se présente sous la forme d'un fond de teint, d'une laque à lèvres ou d'un rouge à lèvres.

**29.** Composition selon l'une quelconque des revendications précédentes telle que le rapport en poids de la charge « soft-focus » sur la fraction non volatile de la phase grasse est compris entre 30 :70 à 50 :50.

**30.** Composition selon l'une quelconque des revendications précédentes se présentant sous forme fluide ou solide, à l'exclusion des formes poudres.

**31.** Composition selon l'une quelconque des revendications précédentes se présentant forme fluide, sous forme pâteuse, sous forme semi-solide, et sous forme solide à l'exclusion des formes poudres.

**32.** Procédé non thérapeutique de maquillage de la peau, caractérisé par le fait que l'on applique sur la peau une composition telle que définie à l'une quelconque des revendications 1 à 31.

**33.** Utilisation d'au moins une charge « soft-focus » et d'au moins une substance fluorescente dans une composition comprenant au moins une phase grasse comprenant une fraction non volatile dans le but de conférer à la peau un effet soft-focus amélioré.

**34.** Utilisation d'au moins une charge « soft-focus » et d'au moins une substance fluorescente pour la préparation d'une composition comprenant au moins une phase grasse comprenant une fraction non volatile, dans le but de conférer à la peau un effet soft-focus amélioré.

Office européen des brevets

**RAPPORT DE RECHERCHE EUROPEENNE**

Numéro de la demande
EP 00 40 3044

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 0 087 060 A (A.W.FABER-CASTELL) 31 août 1983 (1983-08-31)<br>* exemples 1-4 * | 1-7, 10-13, 15, 19-23, 26-28, 30-32 | A61K7/48 |
| A,D | EP 0 370 470 A (ESTEE LAUDER INC.) 30 mai 1990 (1990-05-30)<br>* colonne 4, ligne 18 - colonne 7, ligne 17 * | 1-7, 10-14, 17-20, 22,23, 26-28, 30-32 | |
| A,D | FR 2 741 261 A (GENERAL ELECTRIC COMPANY) 23 mai 1997 (1997-05-23)<br>* revendications 11,12 * | 1,15 | |
| A | DATABASE WPI<br>Week 199133<br>Derwent Publications Ltd., London, GB;<br>AN 1991-242246<br>XP002142983<br>& JP 03 157315 A (TANAKA),<br>5 juillet 1991 (1991-07-05)<br>* abrégé * | | |

DOMAINES TECHNIQUES RECHERCHES (Int.Cl.7)

A61K

Le présent rapport a été établi pour toutes les revendications

| Lieu de la recherche | Date d'achèvement de la recherche | Examinateur |
|---|---|---|
| BERLIN | 15 février 2001 | Alvarez Alvarez, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 00 40 3044

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-02-2001

| Document brevet cité au rapport de recherche | Date de publication | Membre(s) de la famille de brevet(s) | Date de publication |
|---|---|---|---|
| EP 87060 A | 31-08-1983 | DE 3204636 A | 25-08-1983 |
| | | DE 3364460 D | 21-08-1986 |
| | | JP 58198410 A | 18-11-1983 |
| EP 370470 A | 30-05-1990 | CA 2003346 A | 23-05-1990 |
| | | DE 68910477 D | 09-12-1993 |
| | | DE 68910477 T | 07-04-1994 |
| | | JP 2200612 A | 08-08-1990 |
| | | US 5143723 A | 01-09-1992 |
| | | US 5324506 A | 28-06-1994 |
| FR 2741261 A | 23-05-1997 | DE 19646804 A | 22-05-1997 |
| | | GB 2307639 A,B | 04-06-1997 |
| | | JP 9183714 A | 15-07-1997 |
| | | US 5830446 A | 03-11-1998 |
| JP 3157315 A | 05-07-1991 | AUCUN | |

EPO FORM P0460

Pour tout renseignement concernant cette annexe : voir Journal Officiel de l'Office européen des brevets, No.12/82